# EUROPEAN PATENT APPLICATION

(11) **EP 2 818 191 A1**
(43) Date of publication of application: **31.12.2014**
(21) Application number: 13173478.2
(22) Date of filing: 25.06.2013
(51) Int. Cl.: A61M 5/142, A61M 5/158

(54) **Einführinstrument mit Parametereinstellung**

(71) Applicant: F. Hoffmann-La Roche AG, 4070 Basel (CH); Roche Diagnostics GmbH, 68305 Mannheim (DE)
(72) Inventor: Gescheit, Illai, Tel Aviv (IL)
(74) Representative: Rentsch Partner AG

(57) **Abstract**

Disclosed is an inserter for inserting a transcutaneous device (300) into a patient's tissue for an extended time period, the inserter (100) including:
- a moving unit (120), the moving unit (120) being designed to couple to the transcutaneous device (300) and to carry out an insertion movement, the insertion movement displacing the transcutaneous device (300) from an initial distal resting position where a transcutaneous member (310) of the transcutaneous device (300) is retracted behind a proximal contact surface (131) of the inserter (100) into a subsequently maintained proximal resting position where the transcutaneous member (310) projects from the proximal contact surface (131);
wherein the moving unit (120) includes an electronic controlled parameter actuator (121) for setting at least one insertion parameter of the insertion movement,
- an electronic inserter controller (110) for controlling operation of the parameter actuator (121),

wherein the inserter controller (110) and the parameter actuator (121) are operatively coupled for activating the parameter actuator (121) to set the at least one insertion parameter.

Disclosed are further an insert-cradle assembly as well as a method for inserting a transcutaneous device into a patient's tissue

## Description

### Technical field

The present disclosure is direct towards inserters for inserting a transcutaneous device into a patient's tissue for an extended time period, as well as inserter-cradle assemblies comprising an inserter as well as a skin-attachable cradle. Transcutaneous devices that may be inserted by and used in combination with such an inserter include infusion cannulas as typically used for insulin infusion as well as transcutaneous analyte sensors, such as continuous glucose sensors.

### Background, prior art

In the therapy of diabetes mellitus, Continuous Subcutaneous Insulin Infusion (CSII) on the basis of a miniaturized, computer-controlled infusion pump that is carried by a diabetic substantially continuously is a well-established form of therapy. Insulin infusion is carried out via a transcutaneous device in form of an infusion cannula that is placed in the subcutaneous tissue and replaced by the diabetic, a relative or the like (also referred to as "user") every few days in the home environment. The infusion cannula may either fluidically couple to the infusion pump via catheter tubing or may form, together with the infusion pump, a compact unit that is directly arranged at the infusion site and typically adhesively attached to the skin.

The infusion cannula may be sufficiently rigid to pierce the skin and be inserted into the tissue; in this case, the design of infusion cannula is similar to a standard hypodermic syringe cannula and is typically made from medical grade stainless steel. Alternatively, the infusion cannula may be comparatively soft in order to improve the wearing comfort and be made of Teflon® or a similar material. In this case, the infusion cannula as such has the general form of a small tube with a blunt proximal end. Such an infusion cannula is inserted into the tissue together with a rigid insertion needle or - more generally - an insertion sharp that is arranged inside the cannula lumen in snug sliding fit and has a pointed proximal tip that extend beyond the blunt proximal end of the infusion cannula. Following the insertion into the tissue, only the insertion needle is retraced.

In the first years of CSII therapy, transcutaneous placing of the infusion cannula as well as retraction of the insertion needle, if present, was generally done manually. Factors such as psychological concerns, an increasing demand for carrying out the insertion under reproducible conditions that are considered best suited with respect to reliable insertion as well as pain minimization, and user comfort demands resulted in the development of a variety of dedicated inserters that carry out a suited insertion movement as well as subsequent retraction movement of an insertion sharp, if present, upon simply actuating a trigger button, or the like. Exemplary designs of such spring-driven inserters are disclosed, e.g., in the WO 2005/046780 A1, US2004/0133164A1, US 7879010 B2, WO 2004/101071 A2, US 2007/0093754 A1, EP 2173410 A1.

A similar situation as described before with respect to infusion cannulas is given for analyte sensors that are placed in a similar way in the subcutaneous tissue for a number of days. In diabetes therapy, continuous glucose sensors are increasingly used for continuously measuring glucose concentration in the blood or the interstitial liquid of the subcutaneous tissue either in combination with an insulin pump or stand-alone.

In the present disclosure, the insertion of infusion cannulas and/or continuous glucose sensors is exemplarily used as field of application. Inserters according to the present disclosure may be used in the same way in other diagnostic or therapeutic fields; examples are pain therapy or cancer therapy where infusion via a subcutaneous cannula may be done as well. The transcutaneous device may further be a combined device that integrates multiple functions and integrates, e.g. an infusion cannula and a glucose sensor in an integral unit.

### Summary of disclosure

It is an overall objective of the present disclosure to provide improved inserters for inserting a transcutaneous device into a patient's tissue for an extended time period. According to one aspect, the present disclosure is directed to an inserter.

The transcutaneous device includes a transcutaneous member as the element that actually pierces the skin and is inserted into the tissue. The transcutaneous device may further include, distal to the transcutaneous member, a body member that is not inserted to be inserted into the tissue but to rest on or close to the skin outside the patient's body. The body member is typically made from plastic and may serve as handle and provide electric, fluidic and/or mechanical interfaces. "Inserting a transcutaneous device" is accordingly to be understood as inserting at least part of the transcutaneous member into the tissue such that the transcutaneous device can operate as intended.

An inserter according to the present disclosure may include a moving unit, the moving unit being designed to couple to a transcutaneous device and to carry out an insertion movement. The insertion movement displaces the transcutaneous device from an initial distal resting position where a transcutaneous member of the transcutaneous device is retracted behind a proximal contact surface of the inserter into a subsequently maintained proximal resting position where the transcutaneous member projects from the proximal contact surface.

Here and in the following, the terms "proximal" and "distal" are used with reference to the skin surface during operation. The skin surface generally substantially corresponds to the proximal reference surface of the inserter.

The moving unit may include an electronically controlled parameter actuator for setting at least one insertion parameter of the insertion movement. The inserter may further include an electronic inserter controller for controlling operation of the parameter actuator. The inserter controller and the parameter actuator may be operatively coupled for activating the parameter actuator to set the at least one insertion parameter. For establishing the coupling to the transcutaneous device, the moving unit includes, in some embodiments, a transcutaneous device coupler. In such embodiments where the transcutaneous device is mechanically locked to a transcutaneous device coupler during the in insertion movement, a release mechanism is favourably present to unlock the transcutaneous device from the transcutaneous device coupler at the end of the insertion movement.

The moving unit generally includes an insertion mechanism with an insertion actuator for carrying out the insertion movement. In some embodiments, the insertion actuator is an electrically powered actuator such as a standard DC motor or stepper motor or a solenoid actuator. For realizing the typically linear insertion movement, a motor as insertion actor may, e.g. act via a gear on a toothed bar. In embodiments with an electrically powered insertion actuator, no separate parameter actuator may be required but the at least one insertion parameter may be controlled via the drive power, in particular the current and/or voltage provided to the actuator as a function of time. For such embodiments, the parameter actuator may accordingly be integral with the insertion actuator. In alternative embodiments, the insertion mechanism is generally designed in the same way as typical state-of-the-art inserters, using a drive spring or a system of drive springs as insertion actuator and storage for providing the energy that is required for the insertion movement. In such embodiments, a dedicated electrical parameter actuator, e.g. in form of one more motor(s) or solenoid(s), may be required. In accordance with the overall design of the insertion mechanism, the parameter actuator may, e.g., control the bias of the drive spring(s) and/or frictional damping elements. In further embodiments including one or more drive spring(s), the springs are loaded, i.e. energized by the user prior to application. Alternatively, the spring(s) may be loaded via an electrically powered actuator which may at the same time serve as parameter actuator. A motor may, for example, be provided to load the spring(s) to store a selected amount of spring energy as defined by an insertion parameter. In further embodiments, the moving unit is hydraulic or pneumatic, as will be discussed in more detail in the context of exemplary embodiments. For all inserter designs, the force or energy that is required for the insertion movement to be carried out is stored by the inserter e.g. in form of spring compression or gas compression energy or electrical energy and is transmitted onto the transcutaneous device when carrying out the insertion movement.

In embodiments including an electrically driven insertion actuator, triggering of the insertion, i.e. initiation of the insertion movement to be carried out, is typically done by energizing the insertion actuator. In embodiments including a spring-powered insertion actuator, the insertion is typically triggered by releasing the biased and thereby energized spring(s), using, e.g. a latch arrangement. Releasing may be achieved via a trigger actuator, such as a solenoid. For spring-powered devices, a purely mechanical trigger mechanism may be provided or, alternatively, an electrical powered trigger mechanism with a trigger actuator, e.g. a solenoid.

The inserter controller is typically realized as semiconductor-based electronic control circuitry, including the required control logics as well as drive circuitry for driving the parameter actuator.

In some embodiments, the inserter includes an insertion feedback sensor in operative coupling with the moving unit and the insertion controller. The insertion feedback sensor is arranged to determine at least one measure that is related to the insertion movement and provides feedback to the inserter controller. Such feedback may especially be used for one or more of diary keeping, insertion supervision and closed-loop control of the insertion.

In some embodiments of the inserter, the at least one insertion parameter includes at least one of (i) a displacement velocity, (ii) a displacement velocity versus time profile, (iii) a displacement distance, and (iv) an insertion angle of the transcutaneous device relative to a reference surface.

The reference surface may especially be given by the proximal contact surface or by another surface of the inserter that is fixed relative to the proximal contact surface. The insertion angle is accordingly the angle with which the transcutaneous device is inserted into the tissue. Controlling the insertion angle as insertion parameter in particular allows usage of the same inserter for inserting transcutaneous devices that require different insertion angles, e.g. perpendicular to the skin or at a small angle of, e.g. 10° ... 30° relative to the skin surface.

Controlling the displacement velocity and/or the velocity versus time profile in particular allows - in an aspect - adoption for different types of transcutaneous devices that should, in dependence of their specific design - be inserted with different insertion velocities, such as rigid steel infusion cannulas, soft Teflon® cannulas and glucose sensors. In another aspect it allows adaption for different patients with their individual insertion preferences for pain minimization. A suited or preferred insertion velocity may further depend on the insertion site, i.e. the location on the patient's body where the transcutaneous device is inserted and is typically different, e.g. for the abdomen region and a thigh.

Controlling the displacement distance allows controlling the insertion depth of the transcutaneous member into the tissue. Due to the inter-patient variability, as reflected, e.g., by different body-mass-indices (BMIs), infusion cannulas of different length currently need to be provided, with the cannula length, i.e. the length of the transcutaneous member that is inserted into the tissue, varying, e.g. between 8 mm and 18 mm. Controlling - via the displacement distance - the insertion depth allows providing only a single or a reduced number of transcutaneous device types and further allows selecting an insertion depth that precisely meets the patient's individual requirement, rather than being bound to a fixed set of available cannula lengths. It further allows modifying the insertion depths due to a change, e.g. of the BMI.

In some embodiments, the at least one insertion parameter is a parameter set of multiple insertion parameters.

In some embodiments, the inserter includes a transcutaneous device sensor, wherein the transcutaneous device sensor is part of or operatively coupled to the inserter controller. A transcutaneous device sensor may be provided to detect whether or not a transcutaneous device is present and operatively coupled with the moving unit. In an embodiment, the transcutaneous device sensor may be an electrical (micro) switch that is actuated by establishing coupling of moving unit and transcutaneous device. In further embodiments, a more sophisticated transcutaneous device sensor is present, e.g. in form of an RFID reader for wireless operative coupling to an RFID tag of the transcutaneous device.

In some embodiments including a transcutaneous device sensor, the inserter is designed to enable the insertion movement to be carried out only if the transcutaneous device sensor indicates the presence of the transcutaneous device in operative coupling to the inserter. The transcutaneous device sensor accordingly acts as safety device that prevents accidentally triggering the insertion without transcutaneous device. Additionally or alternatively to the transcutaneous device sensor, a skin-contact sensor or a cradle engagement sensor may be provided to enable the insertion movement to be carried out only if the inserter is properly placed in contact with the patient's skin, or is attached to a cradle, respectively.

In some embodiments including a transcutaneous device sensor, the inserter controller is designed to control the parameter actuator in accordance with information provided by the transcutaneous device sensor. For this type of embodiment, the optional transcutaneous device sensor is designed not only to detect the presence of the transcutaneous device, but also to read from the transcutaneous device information indicative of the at least one insertion parameter, thus allowing an automatic setting of the at least one insertion parameter in dependence of the transcutaneous device. If the transcutaneous device sensor is an RFID reader as described before, the information may be stored in an RFID tag of the transcutaneous device. Further alternatives are a number of electrical (micro) switches that are arranged to be selectively actuated, e.g., by corresponding coding projections on the transcutaneous devices, or contacts that are designed to galvanic couple with corresponding conductive pads on the transcutaneous device, or the like. The information indicative of the at least one insertion parameter, e.g. an insertion velocity, may be directly stored by the transcutaneous device and read by the transcutaneous device sensor. Alternatively, the information may be identification information identifying the type of transcutaneous device, with the corresponding insertion parameter(s) being stored by a parameter memory of the inserter.

In some embodiments, the inserter controller includes a parameter memory for storing a preset value for the at least one insertion parameter. In some embodiments including a parameter memory, the parameter memory is designed to store a set of alternative preset values for the at least one insertion parameter. In some of those embodiments, alternative values are stored for different types of transcutaneous device, with the selection of the at least one parameter being made manually by a user, automatically based on information provided by a transcutaneous device sensor, or a combination. Additionally or alternatively, different preset values may be stored, e.g. for different insertion sites.

In some embodiments, the inserter controller is operatively coupled or operatively coupable to a user interface and is designed to receive from the user interface information indicative of the at least one insertion parameter. Via the user interface, the insertion parameter(s) may be directly entered, e.g. as numeric value, using one or more rotary knob(s), scroll bar(s) etc. Alternatively, the user interface may be designed for a more qualitative entry and provide options such as "high", "medium", and "low" insertion velocity.

In some embodiments, the inserter controller is operatively coupled or operatively coupable to a user interface and is designed to receive from the user interface user identification information and to enable the insertion movement to be executed only after verification of a user identity. Verification may be carried out, e.g. by requesting the entry of a PIN code from the user. In embodiments where alternative preset values are stored in a parameter memory, a different PIN may be foreseen for the different alternatives. In further embodiments, more sophisticated arrangements are provided for user identification such as a finger print sensor.

In some embodiments, the inserter is designed to operatively wirelessly couple to a physically separate remote controller.

For embodiments where the insertion is triggered via an electric signal, the inserter may be designed to receive a trigger signal that is generated remotely by the remote controller. This type of embodiment is particularly useful if the insertion site is not easy accessible such that operating a trigger button or the like directly at the insertion site is difficult. Alternatively or additionally, a remote controller may be provided for further control operations, in particular for entering and/or selecting insertion parameters.

The remote controller may be a dedicated device for exclusive use in combination with the inserter. Alternatively, it may be a multi-purpose device such as a smart phone. In further embodiments, the remote controller is part of a therapy management device which may, e.g. include additional functional and/or structural units, such as a remote controller for an infusion device, a glucose meter, etc.

In some embodiments, the moving unit is designed to displace, when carrying out the insertion movement, an insertion sharp, such as an insertion needle, along with the transcutaneous device and, subsequent to the insertion movement, to carry out a retraction movement, the retraction movement displacing the insertion sharp from the proximal resting position to a distal resting position where the insertion sharp is enclosed by the inserter with the proximal resting position being maintained by the transcutaneous device.

Inserting an insertion sharp together with the transcutaneous device is especially required for the insertion of soft infusion cannulas. In such embodiments, the insertion sharp is typically realized by a rigid pointed needle that is initially arranged in snug sliding fit inside the lumen of the soft cannula. An insertion sharp is further required for inserting some types of glucose sensors.

The moving unit of such embodiments is favourably designed such that the coupling with the transcutaneous device is released at the end of the insertion movement while coupling with the insertion sharp is maintained at the end of the insertion movement or only established at the end of the insertion movement for retracting the insertion sharp.

In dependence of the type of insertion actuator and the overall kinematic design of the moving unit, it may be possible to use the insertion actuator also for the retraction movement. This may be the case especially if the insertion actuator is reversible, such as an electric motor. In alternative designs, a separate retraction actuator is provided, e.g. in form of a retraction spring. While controlling parameters of the retraction movement is not required in many cases, it is generally well possible in an analogous way to the insertion movement and encompassed by the present disclosure.

In further embodiments of inserters that are designed for inserting an insertion sharp along with the transcutaneous device, the inserter is not designed for automatic retraction of the insertion sharp; instead, the insertion sharp is retracted manually by the patient after removal of the inserter. While being somewhat less comfortable, the inserter design is simplified.

In some embodiments, the inserter includes a history memory in operative coupling with the inserter controller. Such typically non-volatile history memory may be designed to store relevant information with respect to insertions that are carried out with the inserter, such as date, time, and insertion parameters. Such history memory may further store data that relates to the operation of inserter, such as error messages.

In some embodiments, the inserter includes a further moving unit, the further moving unit being designed to couple to a further transcutaneous device and to carry out a further insertion movement, the further insertion movement displacing the further transcutaneous device from a further initial distal resting position where a further transcutaneous member of the further transcutaneous device is retracted behind the proximal contact surface of the inserter into a subsequently maintained further proximal resting position where the further transcutaneous member projects from the proximal contact surface. In contrast to a combined device that integrates the functionality of more than one transcutaneous device and may be inserted in a single step using a single moving unit, the further transcutaneous device is distinct and physically separate from the transcutaneous device and requires separate insertion.

An inserter according to this type of embodiment accordingly allows, via the moving unit and the further moving unit, the insertion of both a transcutaneous device and a further transcutaneous device. Such an inserter is favourable, e.g. for both insulin infusion and glucose measurement if the infusion cannula and the glucose sensor shall be placed in the tissue in some distance in order to avoid functional interdependence.

In dependence of the design of the further transcutaneous device, the further moving unit may be designed in generally the same way as the moving unit, or according to another principle. A further parameter actuator of the further moving unit may operatively couple to the inserter controller in the same way as the moving unit, thus allowing the inserter controller to control at least one parameter of the further insertion movement. Insertion parameter setting may be separate for the moving unit and the further moving unit or may be coupled, resulting in a common insertion parameter influencing operation of both the moving unit and the further moving unit.

In some embodiments, such an inserter may be designed such that carrying out the insertion movement and the further insertion movement is synchronized, i.e. that the insertion movement and the further insertion movement is carried out simultaneously or with a given or selectable time delay. This allows carrying out both insertions while only once inducing the unavoidable pain or discomfort that is generally associated with skin piercing. Alternatively, the inserter is designed for separate and independent triggering of the insertion movements.

In some embodiments including a further moving unit, the moving unit and the further moving unit are functionally and structurally separate. Alternatively, however, they share common units, such as a common insertion actuator and/or a common parameter actuator or may share a common moving unit.

While this document is largely focuses of embodiments that are designed for the insertion of a single transcutaneous device, the principles and embodiments may be modified for embodiments for the insertion of a further transcutaneous device.

In some embodiments, the inserter includes a cradle interface, the cradle interface being designed to temporarily locking attachment of the inserter, for the insertion movement, to a distal side of a skin attachable cradle. Due to a temporary attachment of the inserter to a previously skin-attached cradle, the insertion can be carried out without the patient's hands holding or generally having any contact with the inserter during the insertion. This is especially favourably in embodiments where actually triggering the insertion is done via a remote controller. The cradle interface is designed to maintain, after attachment to the cradle, the inserter in place without any further support being required. The cradle interface further includes aligning means for ensuring proper alignment of cradle and inserter. Following the insertion, the inserter is favourably removed by the user with the cradle staying skin-attached. In this context, "releasable" means that the attachment can be released by the user in an easy way, e.g. by pressing one or more release buttons, favourably without requiring any further tools and in particular without damaging either of the inserter or the cradle.

According to a further aspect, the present disclosure is directed towards an inserter-cradle assembly. The inserter-cradle assembly may include an inserter as generally described before and further below in the context of exemplary embodiments, and a cradle. The cradle has an adhesive proximal side for adhesive attachment to a patient's skin and a distal side, the distal side having a device interface. The device interface is designed for releasable locking attachment of the inserter and for subsequent locking attachment of a medical device. The device interface further includes a transcutaneous device engagement structure, the transcutaneous device engagement structure being arranged such that the transcutaneous device is, at the end of the insertion movement, locked with the transcutaneous device engagement structure in the proximal resting position such that the transcutaneous member projects in proximal direction from the proximal side of the cradle.

Because the transcutaneous device locks with the transcutaneous device engagement structure, the inserter can, subsequent to the insertion, be removed. According to this aspect of the present disclosure, the cradle is accordingly used for first temporarily attaching the inserter and subsequently attaching the medical device to the patient's skin.

The medical device may, e.g. be a skin-mountable infusion device or a blood glucose monitoring device. An exemplary combination of a glucose senor and a cradle is disclosed in the US 2004/0133164 A1; an exemplary combination of an infusion device and a cradle is disclosed in the WO 2009/06635 A2. After attaching the medical device to the cradle, the medical device operatively couples to the transcutaneous device. In case of the medical device being an infusion device and the transcutaneous device being an infusion cannula, such coupling may be fluidic. In case of the medical device being a blood glucose monitoring device and the transcutaneous device being a blood glucose sensor, such coupling may be electric or fluidic.

According to a still further aspect, the present disclosure is directed toward a method for inserting a transcutaneous device into a patient's tissue for an extended time period. The method may include carrying out an insertion movement, the insertion movement displacing the transcutaneous device from an initial distal resting position where a transcutaneous member of the transcutaneous device is retracted behind a proximal contact surface of the inserter into a proximal resting position where the transcutaneous member projects from the proximal contact surface. The method further includes, at the end of the insertion movement, maintaining the proximal resting position of the transcutaneous device. The method may further include setting at least one insertion parameter of the insertion movement via an electronically controlled parameter actuator.

An inserter according to the present disclosure may especially be used for carrying out a method for inserting a transcutaneous device according to the present disclosure. Therefore, all disclosed embodiments of inserters as described before as well as in the following in the context of specific embodiments disclose, at the same time, corresponding embodiments of a method for inserting a transcutaneous device, and vice verse, even if only either of an inserter embodiment or an insertion method embodiment is explicitly described for the sake of conciseness.

### Exemplary embodiments

In the following, exemplary embodiments are discussed in greater detail with additional reference to the figures.
Figure 1 shows an exemplary inserter together with an exemplary transcutaneous device and an exemplary cradle.
Figure 2 shows an exemplary control structure of an inserter together with associated units.
Figure 3 shows an exemplary remote controller with a graphical user interface.
Figure 4 shows an exemplary remote controller with a further graphical user interface.
Figure 5 shows exemplarily shows the application of an inserter, a cradle and a remote controller.

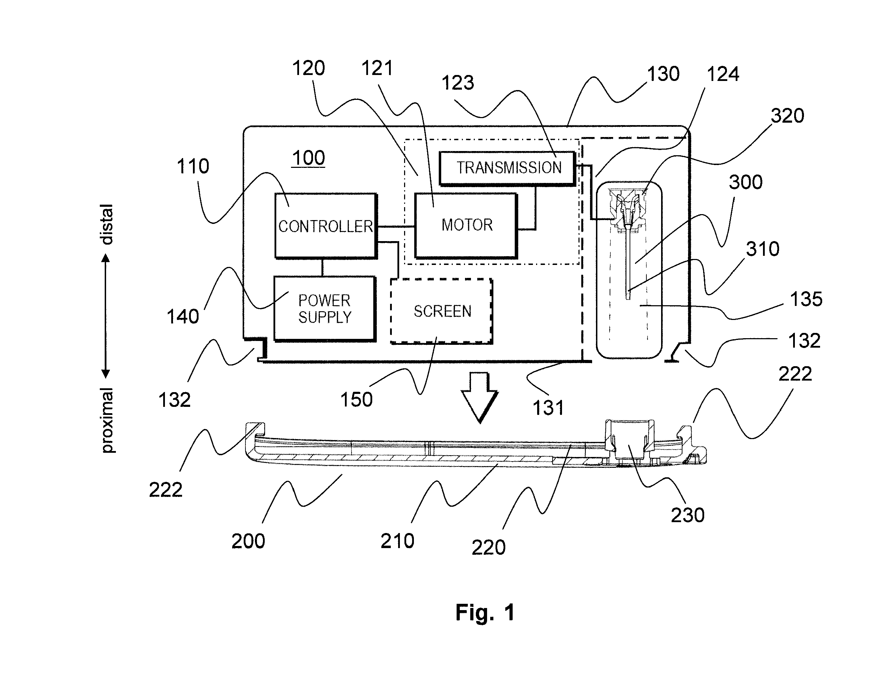

Figure 1 shows an exemplary inserter 100 together with a cradle 200 and a transcutaneous device 300 in a schematic combined structural and functional view.

The transcutaneous device 300 which is as such not part of the inserter 100 but is shown as received by the inserter 100 includes a transcutaneous member 310 and a body member 320. Exemplarily, the transcutaneous device 300 is shown as infusion cannula with the transcutaneous member 310 being typically made from medical grade stainless steel and having a pointed proximal end, while the body member 320 is typically made from plastics.

The inserter 100 includes an inserter controller 110, a moving unit 120, an inserter housing 130, a power supply 140 and an optional screen 150.

The inserter controller 110 is generally based on semi-conductor circuitry and in particular one or more micro controllers, ASICS or the like as well as peripheral circuitry. The inserter controller 100 is further designed for wireless operatively coupling with a remote controller (not shown) that may be used both for general operation of the inserter and in particular parameter setting, and for triggering the insertion. In a variant, a user interface for parameter setting and/or triggering the insertion may be provided additionally or alternatively as part of the inserter 100.

The moving unit 120 is exemplary shown as pneumatic moving unit with a motor 121 which acts on a miniaturized air pump 123, referred to as transmission. The motor 121 is driven and controlled by power circuitry which is included in inserter controller 110. Air pump 123 is coupled via pneumatic line 124 to the inner surface of a guiding channel 135 as will be described further below.

The housing 130 is typically made of plastic and has a proximal contact surface 131 that is designed for attachment to the cradle 200. At the proximal contact surface 131, the housing 130 has a cradle interface in form of recesses 132 the function of which will be described further below.

The inserter 100 further includes a power supply 140 that is typically realized by a rechargeable or non-rechargeable battery and provides power to the inserter controller 110 and to the motor 121. In the embodiment of Figure 1 the inserter 100 further includes an optional screen 150 in operative coupling with the inserter controller 110 for providing user information. While not shown, the inserter 100 may, additionally or alternatively to screen 150, include further indication elements, such as an acoustic transducer, for providing user feedback.

The cradle 200 has a proximal side that is coated or otherwise covered with skin-compatible adhesive (not referenced) for releasable attachment to a patient skin (not shown). The cradle 200 further has an adjacent distal side 220 with a device interface for releasable locking attachment of the inserter 100 and subsequent releasable or non-releasable locking attachment of a medical device. The device interface is exemplarily shown as latches or protrusions 222. The cradle further has a transcutaneous device engagement structure 230 is arranged on the distal side 220 and is designed to receive and lock body member 320 at the end of the insertion movement such that the transcutaneous member 310 projects in proximal direction from the proximal side 210 of the cradle 200.

For carrying out the insertion, the inserter controller 110 controls powering of the motor 121, resulting in air being displaced into the guiding channel 135 distal of the transcutaneous device 300, such that the transcutaneous device 300 is forced in proximal direction from the shown distal resting position into the proximal resting position via air pressure. Therefore, the guiding channel 135 is designed to receive the transcutaneous device 300 with a sliding but snug and largely air-tight fit. The inserter 100 may include a transcutaneous device holder (not shown) that maintains transcutaneous device 300 in the distal resting position prior to the insertion movement. During the insertion movement, the transcutaneous device 300 may move freely and in a floating way inside guiding channel 135. Guiding members may be provided on the inner side of guiding channel 135 and on the transcutaneous device 300, in particular on the body member 320, for ensuring correct orientation of the transcutaneous device 300 during the insertion movement. Such guiding members may, e.g., be realized by mating concave and convex projections on the guiding channel 135 and the body member 320. Alternatively, the guiding channel 135 and the body member 320 may be dimensioned to ensure a snug sliding fit. In further embodiments, a dedicated transcutaneous device coupler is provided as part of the moving unit 120 and couples to the transcutaneous device during the insertion movement; in such an embodiment, the transcutaneous device coupler is designed to release the coupling at the and of the insertion movement.

At the end of the insertion movement, i.e. in the proximal resting position, body member 320 is seated in and locked by the transcutaneous device engagement structure 230.

In the embodiment of Figure 1, the displacement velocity is the sole variable insertion parameter and is controlled via operation of motor 121. In the embodiment of Figure 1, both the insertion actuator as well as the parameter actuator are accordingly realized by motor 121 in an integral way.

The inserter of Figure 1 may be modified in a number of ways, illustrated by the following examples. The moving unit 120 may operate hydraulic rather than pneumatic; in this case, transmission 123 includes a hydraulic pump rather than an air pump and a liquid reservoir is additionally present.

The moving unit may 120 may further be realized in a generally different way. For example, motor 121 may act on the transcutaneous device 300 via mechanical coupling, using e.g. a toothed gear and toothed bar arrangement. Like in the before-mentioned variants, both the insertion actuator and the parameter actuator are realized by motor 121 in an integral way. This type of embodiment further has the particular advantage that, via the voltage and/or current provided to motor 121, the insertion movement, namely the displacement versus time, may be controlled precisely and in a wide range.

In still further designs, the moving unit 120 is generally spring powered. The moving unit may be generally designed in a way as generally known from spring-powered prior art inserters, with a parameter actuator interacting with the insertion spring(s) as described in the general description.

In a further variant of a pneumatic moving unit, a gas pressure reservoir is provided, e.g. in form of a gas cartridge, comprising, e.g., carbonic acid. The gas cartridge couples via a pneumatic line with the guiding channel as discussed before. For this type of embodiment, the parameter actuator may be realized as electrically actuated control valve that is fluidically arranged between the gas cartridge and the guiding channel. Instead of a gas cartridge, a combination of a miniaturized compressor and a pressurized air vessel may be present.

The transcutaneous device 300 may further be designed to include a soft transcutaneous member 310 and additionally include an insertion sharp that may be retracted manually following the detaching of the inserter 100 from the cradle 200 after the insertion or via a retraction spring (not shown) of the inserter 100, as generally known in the art.

The inserter 100 may further be designed to be used stand-alone, i.e., without cradle-unit 200. Such an embodiment may be used for inserting a transcutaneous device that itself includes a skin-adhesive pad, with the skin adhesive pad being placed on the skin at the end of the insertion movement in the proximal resting position. In such a stand-alone inserter, the cradle interface 132 may not be present.

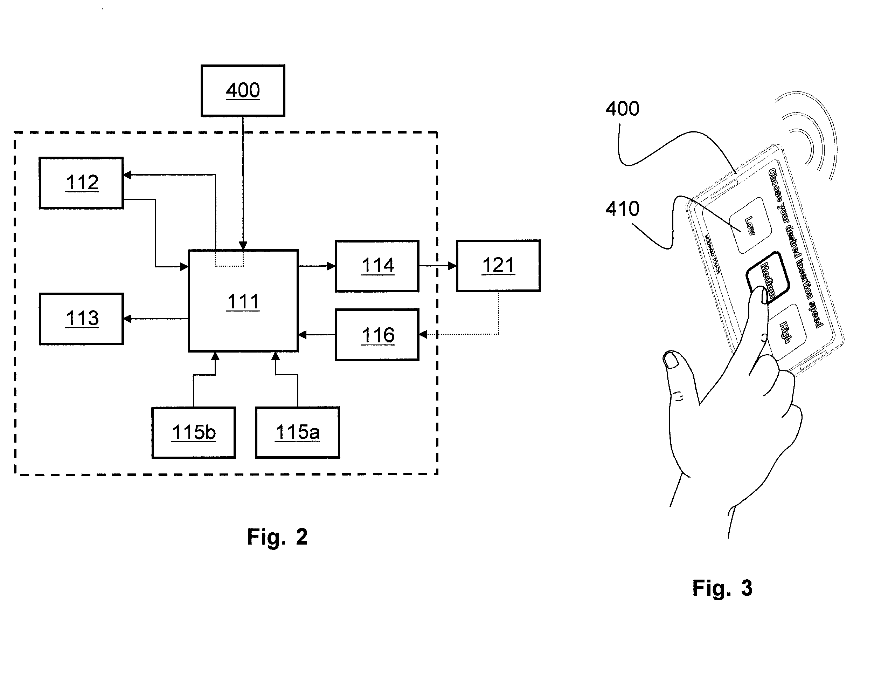

Figure 2 shows an exemplary design of a control structure and in particular of an inserter controller 110 and associated units in a schematic functional view. The design of the inserter may, e.g. correspond to the example as described with reference to Figure 1, including the described modifications and variants.

The inserter controller 110 includes a central controller 111 that is typically realized as ASIC micro controller or the like. Inserter controller 110 includes a parameter memory 112, a history memory 113, and power circuitry for controlling and powering motor 121 as parameter actuator and insertion actuator. Some or all functional units may be structurally fully separate or fully or partly integral with the central controller 111.

The inserter controller 110 includes a parameter memory 112 that exemplarily stores a set of alternative preset values for the at least one insertion parameter. Here it is assumed that only one insertion parameter, namely the displacement velocity is directly controlled by the inserter controller 110. Further or other insertion parameters may be controlled as well.

A user interface is provided by a physically separate remote controller 400 in wireless operative coupling with the inserter controller 110 and in particular its central controller 111. Via the central controller 111, the remote controller 400 operatively couples with the parameter memory. For the moment, it is exemplarily assumed that the parameter memory stores three preset displacement velocities, referred to as "Low", "Medium" and "High". Another number of preset displacement velocities, in particular a larger number, may be stored as well. The velocities may be factory-set, in which case an individual user modification may or may not be possible, or may be generally set by the user. In this exemplary embodiment, remote controller 400 is also used for triggering the insertion, i.e. initiating the insertion movement to be carried out.

Optional history memory 113 is provided in operative coupling with central controller 111. The history memory 113 is favourably non-volatile and designed to store relevant information with respect to insertions that are carried out with the inserter 100, typically including the date and time of day of insertions, as provided by a clock module that may be part of the central controller 111. Favourably, history memory 113 also stores, for each insertion, the value of the at least one insertion parameter as actually used for an insertion. The history memory 113 may further store data that relates to the operation of inserter 100, such as error messages. The data stored in memory 100 is useful for diary keeping purposes of the user as well as for assessing the cause of a number of therapy complications as well as device diagnosis. It is noted that the history memory 113, if present, is not necessarily an integral part of inserter 100 but may also be, e.g., part of the remote controller 400.

Optional transcutaneous device sensor 115a is provided in operative coupling with central controller 111. The transcutaneous device sensor 115a is exemplarily considered as binary switching sensor that detects the presence of the transcutaneous device and prevents the insertion to be triggered, i.e. the motor 121 to be actuated, without presence of the transcutaneous device. Alternatively, however, the transcutaneous device sensor 115 may be designed to read from the transcutaneous device the at least one insertion parameter or information that is related to the at least one insertion parameter as explained before in the context of the general disclosure.

Optional cradle engagement sensor 115b is provided in operative coupling with central controller 111. The cradle engagement sensor 115b is a binary sensor that detects whether the inserter 100 is attached to a cradle and prevents the insertion to be triggered, i.e. the motor 121 to be actuated, without such attachment. The cradle engagement sensor 115b may, e.g., be realized as micro switch or reed switch. In embodiments of the inserter 100 that are designed to be directly placed on the skin without cradle, the sensor 115b may be a skin contact sensor that may, e.g., also be realized as mechanically actuated micro switch, as capacitive switch, or the like.

Optional insertion feedback sensor 116 is provided in operative coupling with the moving unit 120 and the central controller 111. The insertion feedback sensor 116 determines at least one measure that is related to the insertion movement and provides feedback to the inserter controller 110 and in particular the central controller 111. The feedback may be used for either or more of monitoring the insertion movement, detecting insertion faults and closed-loop control of one or more insertion parameter(s). In an example, the insertion feedback sensor 116 is a rotary encoder that couples to a drive shaft of the motor 121. In case of a pneumatic or hydraulic moving unit as exemplarily shown in Figure 1, the insertion feedback sensor 116 may include one or more pressure sensors. In further embodiments, the insertion feedback sensor 116 is or includes a position sensor that determines the displacement position of the transcutaneous device or an associated component of the moving unit 120, such as a transcutaneous device holder, along the axis of the insertion movement. In further embodiments, the insertion feedback sensor 116 is or includes one or more binary displacement position sensor(s) such as light barrier(s) that is/are actuated by the transcutaneous device 300 or an associated component of the moving unit 120 at one or more displacement position(s). Alternatively or additionally to providing a dedicated insertion feedback sensor 116, the electric control and drive signal provides information to the moving unit, such as the current through the motor 121 or the induced counter voltage of the motor 121.

Figure 3 exemplarily shows a remote controller 400 that may be used in combination with an inserter 100, such as an inserter as illustrated in Figures 1, 2 in a schematic view. Remote controller 400 is designed for wireless remote communication with the inserter 100, based, e.g. on the Bluetooth standard, a further general purpose standard or using a proprietary protocol. Exemplary remote controller 400 includes a touch screen 410 as user interface. In Figure 3, the touch screen shows a Graphical User Interface (GUI) for selecting either of a low, medium or high displacement velocity for the insertion of the transcutaneous device 300. Further graphical user interface are typically provided for controlling further operations, such as triggering the insertion.

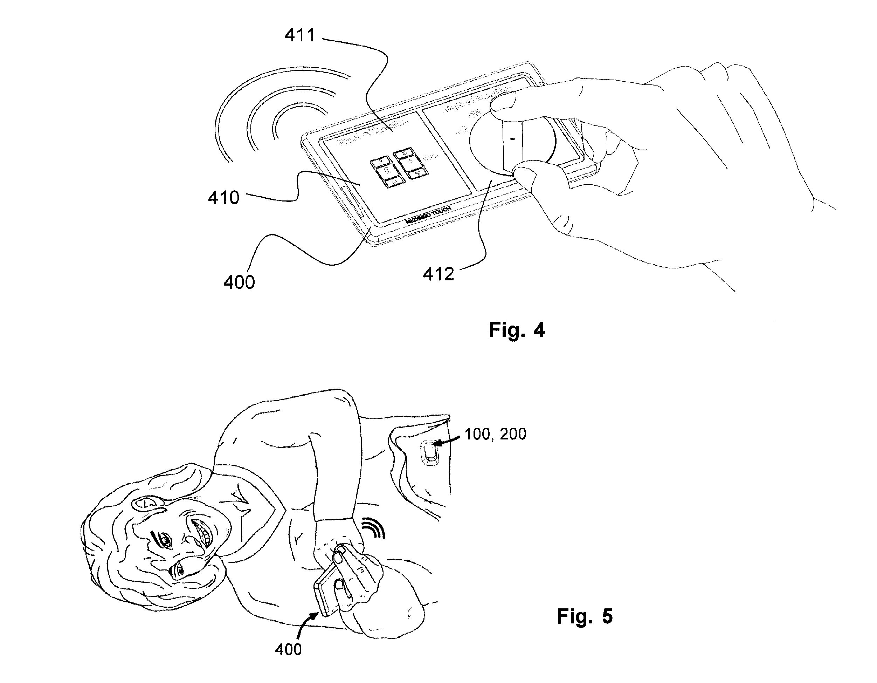

Figure 4 exemplarily shows a remote controller 400 similar to Figure 3 with a GUI for entering the depth of insertion or displacement distance via digit entry in field 411 of the touch screen 410 and the insertion angle via rotary knob in field 412 of the touch screen 410. An inserter may be designed for controlling all of displacement velocity, depth of insertion/displacement distance and insertion angle as well as optional further insertion parameters. In this case, the GUIs of Figure 3 and Figure 4 as well as further GUIs may be shown sequentially or selectively. Alternatively, only a single or any combination of insertion parameters may be user set. It is further to be understood that the shown relation between insertion parameter and method of entry as represented by the corresponding GUIs is not mandatory and that further GUIs such as touch screen scroll bars may be used as well. In further embodiments, remote controller 400 may additionally or alternatively include one or more discrete input elements such as conventional knobs, push buttons, scroll wheels etc. It is further noted that remote controller 400 may not be designed to only control inserter 100 but may also be a remote controller for an infusion device or a general purpose device, such as a smart phone.

Figure 5 illustrates practical operation of an inserter 100, such as the inserter shown in Figure 1. The inserter 100 is shown in a state where it temporarily attached and locked to a cradle 200 that has previously been adhesively attached to the patient's body, thus forming an inserter-cradle assembly. Alternatively, the inserter 100 and the cradle 200 may be attached to each other prior to skin mounting and attached adhesively attached to the skin as a combined unit. For carrying out the insertion, there is no need for the user to manipulate or even look at the inserter 100, but all steps like setting of insertion parameters and triggering the insertion are varied out via the remote controller 400. Favourably, the user interface of the remote controller 400 also provides feedback to the user, e.g. in form of either or more of a confirmation message that is shown on a display such as a touch screen of remote controller 400, and/or further optical and/or acoustic confirmation signals. Acoustic signals may be provided by an acoustic transducer, such as a miniaturized speaker or buzzer, of the remote controller 400 or of the inserter 100 in operative coupling with inserter controller 110.

Subsequent to the insertion, the inserter 100 is removed and a medical device, e.g. a skin-mountable infusion device or a blood glucose monitoring device is attached and locked to the cradle 200 in operative coupling with the transcutaneous device.

## Claims

1. Inserter (100) for inserting a transcutaneous device (300) into a patient's tissue for an extended time period, the inserter (100) including:
- a moving unit (120), the moving unit (120) being designed to couple to the transcutaneous device (300) and to carry out an insertion movement, the insertion movement displacing the transcutaneous device (300) from an initial distal resting position where a transcutaneous member (310) of the transcutaneous device (300) is retracted behind a proximal contact surface (131) of the inserter (100) into a subsequently maintained proximal resting position where the transcutaneous member (310) projects from the proximal contact surface (131);
wherein the moving unit (120) includes an electronic controlled parameter actuator (121) for setting at least one insertion parameter of the insertion movement,
- an electronic inserter controller (110) for controlling operation of the parameter actuator (121),
wherein the inserter controller (110) and the parameter actuator (121) are operatively coupled for activating the parameter actuator (121) to set the at least one insertion parameter.

2. Inserter (100) according to claim 1, wherein the at least one insertion parameter includes at least one of (i) a displacement velocity, (ii) a displacement velocity versus time profile, (iii) a displacement distance, and (iv) an insertion angle of the transcutaneous device (300) relative to a reference surface (131, 210).

3. Inserter (100) according to either of the preceding claims, wherein the at least one insertion parameter is a parameter set of multiple insertion parameters.

4. Inserter (100) according to either of the preceding claims, including a transcutaneous device sensor (115a), wherein the transcutaneous device sensor (115a) is part of or operatively coupled to the inserter controller (110).

5. Inserter (100) according to claim 4, wherein the inserter (100) is designed to enable the insertion movement to be carried out only if the transcutaneous device sensor (115a) indicates the presence of the transcutaneous device (300) in operative coupling to the inserter (100).

6. Inserter (100) according to either of claim 4 or claim 5, wherein the inserter controller (110) is designed to control the parameter actuator (121) in accordance with information provided by the transcutaneous device sensor (115a).

7. Inserter (100) according to either of the preceding claims, wherein the inserter controller (110) includes a parameter memory (112) for storing a preset value for the at least one insertion parameter.

8. Inserter (100) according to claim 7, wherein the parameter memory (112) is designed to store a set of alternative preset values for the at least on insertion parameter.

9. Inserter according to either of the preceding claims, wherein the inserter controller (110) is operatively coupled or operatively coupable to a user interface (410) and is designed to receive from the user interface (410) information indicative of the at least one insertion parameter.

10. Inserter according to either of the preceding claims, wherein the inserter controller (110) is operatively coupled or operatively coupleable to a user interface (410) and is designed to receive from the user interface (410) user identification information and to enable the insertion movement to be carried out only after verification of a user identity.

11. Inserter (100) according to either of the preceding claims, the inserter being designed to operatively wirelessly couple to a physically separate remote controller (400).

12. Inserter (100) according to either of the preceding claims, wherein the moving unit (120) is designed to displace, when carrying out the insertion movement, an insertion sharp along with the transcutaneous device (300) and, subsequent to the insertion movement, to carry out a retraction movement, the retraction movement displacing the insertion sharp from the proximal resting position to a distal resting position where the insertion sharp is enclosed by the inserter (100) with the proximal resting position being maintained by the transcutaneous device (300).

13. Inserter (100) according to either of the preceding claims, including a further moving unit, the further moving unit being designed to couple to a further transcutaneous device and to carry out a further insertion movement, the further insertion movement displacing the further transcutaneous device from a further initial distal resting position where a further transcutaneous member of the further transcutaneous device is retracted behind the proximal contact surface (131) of the inserter (100) into a subsequently maintained further proximal resting position where the further transcutaneous member projects from the proximal contact surface (131).

14. Inserter (100) according to either of the preceding claims, wherein the inserter (100) includes a cradle interface (132), the cradle interface (132) being designed for temporarily locking attachment of the inserter (100), for the insertion movement, to a distal side (220) of a skin attachable cradle (200).

15. Inserter-cradle assembly, including
- an inserter according to claim 14,
- a cradle (200), the cradle (200) having an adhesive proximal side (210) for adhesive attachment to a patient's skin and a distal side (220), the distal side (220) having a device interface (222), the device interface (222) being designed for releasable locking attachment of the inserter and for subsequent locking attachment of a medical device, the device interface including a transcutaneous device engagement structure (230) the transcutaneous device engagement structure (230) being arranged such that the transcutaneous device (300) is, at the end of the insertion movement, locked with the transcutaneous device engagement structure (230) in the proximal resting position such that the transcutaneous member(310) projects in proximal direction from the proximal side (210) of the cradle (200).
